Europäisches Patentamt

European Patent Office

Office européen des brevets

(19)

(11) Veröffentlichungsnummer: **0 009 072**
**A1**

(12)  # EUROPÄISCHE PATENTANMELDUNG

(21) Anmeldenummer: 79102090.2

(22) Anmeldetag: 25.06.79

(51) Int. Cl.³: **A 61 F 1/00**

(30) Priorität: 24.08.78  DE 2836944

(43) Veröffentlichungstag der Anmeldung: 02.04.80
Patentblatt 80/7

(84) Benannte Vertragsstaaten: **CH FR GB SE**

(71) Anmelder: **SIGRI ELEKTROGRAPHIT GMBH, Werner
von Siemens-Strasse 18, D-8901 Meitingen (DE)**

(72) Erfinder: **Neugebauer, Rainer, Dr., Liebigstrasse 8,
D-7900 Ulm-Lehr (DE)**
Erfinder: **Wolter, Dietmar, Prof. Dr., Ihlendieksweg 30,
D-2070 Grosshansdorf (DE)**

(54) **Alloplastischer Verschluss von Brüchen.**

(57)  Als alloplastisches Material zur Therapie von Brücken
wird ein netzförmiges Gewebe (1) aus Kohlenstofffäden
vorgeschlagen. Der Umfang (2) des Netzes kann saumartig verstärkt sein.

- 1 -

Alloplastischer Verschluß von Brüchen

Die Erfindung betrifft einen netzförmigen alloplastischen Verschluß von Brüchen.

Nach Operationen im Bereich der Bauchorgane kann es zu Brüchen kommen, deren Ursache eine mangelnde Heilung der durchtrennten Gewebeschichten ist. Der Raum zwischen diesen Muskel- und Fascienschichten ist dabei mit Narbengewebe gefüllt, das häufig der mechanischen Beanspruchung nicht standhält. In der Folgezeit kommt es zu einer Aussackung der Bauchwand durch das nachgebende Narbengewebe, die sich zu einer erheblichen Größe entwickeln kann und eine operative Korrektur erfordert. Falls das körpereigene Gewebe zum Verschluß des Defekts nicht ausreicht, verwendet man alloplastische Materialien zur Verstärkung der Bruchverschlußstelle.

Derartige eine Netzform aufweisende Implantate bestehen beispielsweise aus Polyamid, Polyester oder einem anderen faserförmigen Kunststoff. Da das Einwachsverhalten dieser Materialien im allgemeinen nicht ausreichend ist, konnten sich diese Implantate nicht durchsetzen. Es ist weiterhin bekannt, daß lysofilisierte Dura zur Verstärkung eines Bruchverschlusses verwendet wird. Nachteilig bei diesem Verfahren ist jedoch die postoperative Schwächung des Transplantats durch die Resorptions- und Umbauphase, die zu einem erneuten Auftreten des Bruches führen kann.

Der Erfindung liegt die Aufgabe zugrunde, ein alloplastisches Material zur Therapie von Brüchen zu schaffen, welches gegen das Körpergewebe inert ist und die verschlossene Bruchstelle mindestens so lange mechanisch sichert, bis das körpereigene Reparationsgewebe eine ausreichende Festigkeit entwickelt hat.

Die Aufgabe wird erfindungsgemäß durch die Verwendung eines netzförmigen Gewebes aus Kohlenstoffäden gelöst.

Unter dem Begriff "Kohlenstoffäden", die durch Pyrolyse von faserförmigen Naturstoffen oder synthetischen Faserstoffen erzeugt werden, sollen im folgenden ebenfalls Graphitfäden verstanden werden. Kohlenstoffäden sind her-vorragend gewebeverträglich, wie histologische Unter-suchungen zeigten und die Festigkeit der Fäden ist außerordentlich groß. Die Filamente der zur Herstellung des Netzes verwendeten Fäden oder Fadenbündel, deren Durchmesser etwa 6 bis 10 $\mu$m beträgt und damit etwa der Zellgröße entspricht, induzieren die Bildung von gerichtetem kollagenem Bindegewebe. Jede einzelne C-Faser wird dabei von Körperzellen und anderen Gewebeanteilen umhüllt. Dies führt zu einer sicheren Verankerung des alloplastischen Implantats im Gewebe. Im weiteren Heilungsverlauf brechen die Kohlenstoffasern teilweise und werden sukzessiv durch neugebildetes festes kollagenes Bindegewebe ersetzt, das mechanisch ebenfalls hochbelastbar ist und so ein erneutes Auftreten des Bruches verhindert. Das netzförmige Implantat wird zweckmäßig durch Einzelknopfnähte an dem Muskel- oder Fascien-Gewebe befestigt. Dabei ist es von Vorteil, den Umfang des Netzes saumartig zu verstärken, beispielsweise durch Umlegen oder Verkürzen der Gewebebreite und damit ein Ausreißen des Gewebes zu unterbinden. Zur Verbesserung der Scherfestigkeit der Kohlenstoffilamente kann es schließlich vorteilhaft sein, in an sich bekannter Weise die Filamente mit einer Schicht aus Pyrokohlenstoff zu überziehen, um die Zeitspanne bis zum Zerfall der Filamente zu verlängern.

Die Erfindung wird im folgenden anhand einer schematischen Zeichnung beispielhaft erläutert.

1 ist ein netzförmiges Gewebe aus Kohlenstoffäden mit ca. 10.000 Filamenten, dessen Umfang 2 saumartig verstärkt ist. Die Maschenweite des Gewebes beträgt etwa einen Millimeter. Das Gewebe ist durch Einzelknopfnähte 3 mit dem körpereigenen Gewebe 4 verbunden.

0009072

- 1 -

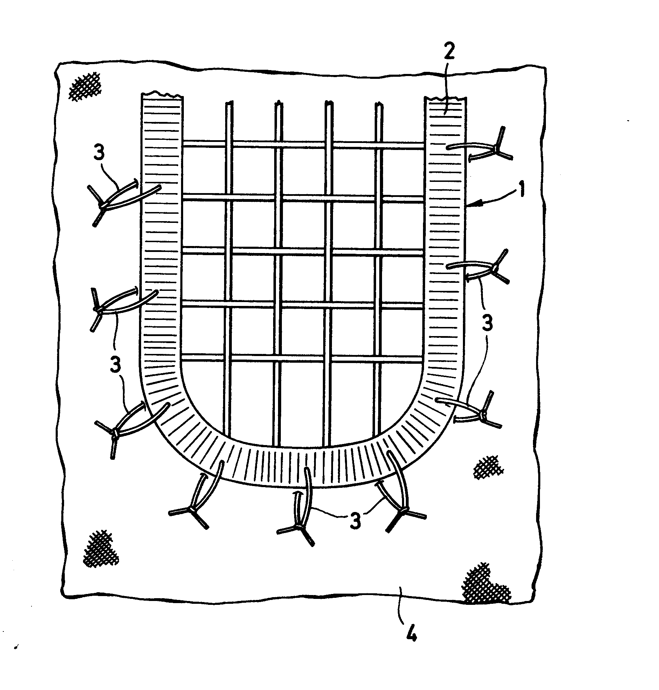

Patentansprüche:

1. Netzförmiger alloplastischer Verschluß von Brüchen,
dadurch gekennzeichnet,
daß das Netz aus Kohlenstoffäden gebildet ist.

2. Alloplastischer Verschluß nach Patentanspruch 1,
dadurch gekennzeichnet,
daß der Umfang des Netzes saumartig verstärkt ist.

3. Alloplastischer Verschluß nach Patentanspruch 1 und 2,
dadurch gekennzeichnet,
daß die Filamente der Kohlenstoffäden mit einer Pyrokohlenstoffschicht überzogen sind.

Europäisches
Patentamt

**EUROPÄISCHER RECHERCHENBERICHT**

0009072
Nummer der Anmeldung

EP 79 102 090.2

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| X | US - A - 3 526 906 (H.G. DE LASZLO) <br> * Anspruch 4; Spalte 1, Zeilen 30 bis 32; Spalte 2, Zeilen 37 bis 41; Spalte 4, Zeilen 64 bis 70; Spalte 5, Zeile 50; Fig. 6 * <br> -- | 1-3 | A 61 F 1/00 |
| | US - A - 3 526 005 (J.C. BOKROS et al.) <br> * Zusammenfassung * <br> -- | 3 | **RECHERCHIERTE SACHGEBIETE (Int. Cl.³)** <br> A 61 F 1/00 |
| A | DE - B - 1 272 488 (P.F.D. TIMMERMANS) <br> * Spalte 4, Zeilen 5 bis 8 und 16 bis 18 * <br> -- | | |
| A | US - A - 3 054 406 (F.C. USHER) <br> * ganzes Dokument * <br> ---- | | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 11-12-1979 | DROPMANN |

EPA form 1503.1 06.78